# EUROPEAN PATENT SPECIFICATION

(11) **EP 3 415 472 B1**
(45) Date of publication and mention of the grant of the patent: **19.02.2020**
(21) Application number: 18177958.8
(22) Date of filing: 15.06.2018
(51) Int. Cl.: C02F 3/28, C12M 1/00, C12M 1/107, C02F 1/44, C02F 11/04, C02F 101/10, C02F 101/16, C02F 103/20

(54) **PROCESS AND INTEGRATED PLANT FOR BIOGAS PRODUCTION FROM ORGANIC WASTES, NUTRIENTS RECOVERY, AND PURIFICATION OF PRODUCED BIOGAS**
VERFAHREN UND INTEGRIERTE ANLAGE ZUR BIOGASERZEUGUNG AUS ORGANISCHEN ABFÄLLEN, NÄHRSTOFFRÜCKGEWINNUNG UND REINIGUNG VON ERZEUGTEM BIOGAS
PROCÉDÉ ET INSTALLATION INTÉGRÉE POUR LA PRODUCTION DE BIOGAZ À PARTIR DE DÉCHETS ORGANIQUES, RÉCUPÉRATION DES NUTRIMENTS ET PURIFICATION DU BIOGAZ PRODUIT

(30) Priority: 16.06.2017 IT 201700067289
(43) Date of publication of application: 19.12.2018
(73) Proprietor: Murgia, Ilaria, 20133 Milano (IT)
(72) Inventor: Murgia, Ilaria, 20133 Milano (IT)
(74) Representative: Ripamonti, Enrico

(56) References cited:
- EP-A1- 2 610 222
- US-A1- 2009 206 028
- US-A1- 2016 023 935
- US-A1- 2016 122 218
- US-A1- 2016 176 768

## Description

### FIELD OF THE INVENTION

The present invention relates to an improved process for biogas production from organic substrates, having an improved degradation efficiency, and to a plant for the actuation of said process. In a preferred embodiment, the present invention further allows the purification of the produced biogas and the recovery of nutrients from the digestate with an improved efficiency with respect to the known processes.

### BACKGROUND

Fermentation of organic substrates operated by suitable consortiums of anaerobic bacteria (bacterial biomass) allows to produce power from agricultural and industrial products and by-products. In particular, starting from the organic substrate, the bacterial biomass produces a mixture of gases (called biogas), through a sequence of known reactions including hydrolysis, acetogenesis and methanogenesis. Biogas is mainly composed by CH₄, CO₂, traces of H₂S, NH₃ and H₂O. At the end of the process, the original organic load is converted into a partially mineralized product (digestate) that contains a higher percentage of inorganic nitrogen and phosphorous with respect to the initial conditions. In addition to biogas, another outcome of the process is therefore the digestate, whose agronomic value has been recently compared to that of chemical fertilizers (Tambone, Adani, 2017).

Since 2000 to date, the number of biogas plants has increased relevantly, pushed by government founding. However, the current concept of biogas plants shows some critical issues, like the large use of "noble" organic substrates like agricultural foodstuffs, a limited efficiency of degradation of the organic matter, the lack of recovery of nutrients (nitrogen and phosphorous) from digestate, the in-site use of biogas with a lowered electrical and thermal efficiency, without any possibility to "store" energy.

In a common configuration, biogas plants are continuous systems and include reactors that are stirred (like continuous stirred tank reactor or CSTR) and thermoregulated at a fixed temperature (usually between 35°C and 45°C), generally using heat produced by an electricity co-generation unit. At each introduction of fresh material, it corresponds an equal extraction of organic substrate (digestant) partially transformed by bacterial biomass. Therefore, part of the fresh product fed to the plant is immediately subtracted from the fermentation volume and consequently does not participate, or partially participates, to the methanogenesis process. Moreover, the continuous removal of digestant product determines an equivalent subtraction of bacterial biomass (so called methanogenic biomass) that is responsible for the fermentation. The bacterial density is the main limiting factor of the plant efficiency. The fermentation speed of the organic load and the related biogas production are directly proportional to the number of bacteria in the fermenter. Consequently, current methods are very inefficient and require huge fermentation volumes to extend the hydraulic residence time (HRT) of the organic load and to allow bacteria multiplication to replace the biomass that is subtracted together with the digestate.

Vegetable substrates, like cornstalks, straws and similar by-products, can be used in addiction to sewages to achieve higher production levels in plants. However, fibrous fractions comprised into the organic load are only partially digested by current plants. Furthermore, when sewages are mainly used, in particular swine and cattle sludges, or other liquid substrates with very low energetic density compared to volume, like milk whey, anaerobic fermenters require huge volumes compared to the production possibilities and it is more difficult to keep them under fixed temperature.

Moreover, nitrogen recovery from organic waste is important in order to use it as a substitute for chemical fertilizers currently adopted, as well as the purification of biogas until it can be classified as "biomethane" and used for transport or introduced into the national network.

State of the art processes for nutrients recovery from sludges are characterized by a considerable engineering plant complexity and by a difficult economic self-sustainability determined by the elevated power consumptions or management costs.

US 2009/206028 A1 discloses a process for the treatment of zootechnical sludges comprising a first anaerobic digestion stage, a phosphorous physical-chemical precipitation stage, a pH readjustment stage by lime addition, an ammonia stripping stage with related recovery, and a stage of purification of the produced biogas by scrubbing.

EP0641296 discloses a process of organic material degradation comprising aerobic and anaerobic digestions carried out in series in dedicated reactors.

WO2010131234 discloses a process of organic wastes treatment comprising a first anaerobic digestion phase and a second aerobic digestion phase; the product of each anaerobic and aerobic digestion phase is filtered and the solid substance is separately returned to the respective digesters. US2016/0122218 A1 and US2016/0023935 A1 describe other process for fermenting organic substrates.

For a really efficient management of nutrients in agriculture, a farms-size plant is fundamental: i.e. a plant that is characterized by a reduced engineering plant complexity, an investment cost proportional to the average size of the farms and - above all - by economic self-maintenance.

The present invention provides a highly improved process for biogas production through the digestion of organic substrate. This organic substrate can include for example: animal sewages, fibrous organic substrates, or industrial or agricultural amylaceous organic materials; in particular it can include: by-products of the processing of cereals, fruit and vegetables and other similar substrates. Preferably, this organic substrate comprises an organic waste material (organic waste), more preferably of zootechnical origin, most preferably swine or cattle sewages. Consequently, the present invention process is also a process for the biological treatment of organic wastes, preferably of zootechnical organic wastes.

The organic substrate digestion occurs under anaerobic conditions, that are conditions of substantial absence of free oxygen or of bound oxygen, in the form of nitrates, nitrites, sulphates, etc., where the final electron-acceptor is the organic substance (Bonomo, 2008). Preferably, oxygen concentration in anaerobic condition is lower than 1 ppm, most preferably is of about 0 ppm.

The present invention overcomes the state of the art critical issues described above, realizing a process and a plant for biogas production with improved degradation efficiency. This improved degradation efficiency is obtained by separating the fibrous and dense fraction of the digestant and re-introducing it in the fermenter, recovering also the methanogenic bacterial biomass from the digestate liquid fraction and reintroducing it in the fermenter, eliminating also a preponderant part of the liquid fraction organic load used as fermenter feed, thus allowing to use even very diluted organic substrates for biogas production.

The invention's process is further advantageous since the alkalinity of the clear fraction produced by the plant as filtered digestate has a value that allows its use, in a preferred aspect of the invention, for purification of biogas from undesired compounds (H₂S, NH₃, CO₂, etc.), thus obtaining a gas with a very high percentage of methane, comparable with a commercial fuel gas. Moreover, in a preferred aspect of the invention's process, a relevant quote of nitrogen included in the fermenting substrate can be captured and destined for the production of commercial salts used as chemical fertilizers; furthermore, a part of the phosphorous included in the fermenting substrate can be extracted under the mineral fractions which is formed during the anaerobic digestion (ashes).

### SUMMARY OF THE INVENTION

The present invention refers to a process as defined in claim 1 for the biogas production from an organic substrate and includes:
i) a first phase of anaerobic digestion of organic substrates by bacterial biomass, with formation of, at least, a partially digested product and a volatile fraction consisting of biogas;
ii) a second phase of separation of the fibrous fraction of digestant and recirculation of the same fibrous fraction into the anaerobic digestion phase;
iii) a third phase of separation of the bacterial biomass present in the digested liquid fraction through microfiltration, and recirculation of that bacterial biomass obtained through microfiltration, back to the anaerobic digestion phase.

Therefore, this process is characterized by a recycle of the fermenting bacterial biomass that, instead of being scattered with the outflow digestate, is separated from digestate by microfiltration process and reintroduced into the fermenter. The anaerobic fermenter bacterial density is thereby considerably increased, allowing a much greater and more efficient degradation of the organic load introduced into the plant with respect to the current methods and, therefore, a greater production of biogas with the same amount of dry matter fed. Moreover, this process is characterized by a similar recycle of the digestant fibrous fraction: in fact, this is separated from the digestate that exits from the fermenter, preferably after shredding, and reintroduced in the same fermenter; in this way, the fibrous fraction has an actual hydraulic residence time much greater than the more rapidly digestible parts of the organic load, determining a much greater degradation thereof.

This separation of the fibrous fraction is essential for the subsequent application of microfiltration: if this separation did not occur, the microfiltration process would undergo rapid malfunctioning and membranes degradation due to the presence of fibrous material. Moreover, eventual unseparated fibrous material, would considerably increase the digestant viscosity, increasing the power consumption of the microfiltration process which works at high liquid flow velocity (3-4 m/s).

Preferably, the invention's process considers the use of the digestate clear fraction, basically free of bacterial biomass, produced after microfiltration (the filtered digestate) to purify the biogas produced by the process, therefore reducing the pollutant gases content and increasing methane percentage. Therefore, according to a preferred embodiment of the present invention, the biogas can be favorably purified, taking advantage of the alkalinity of the filtered digestate obtained from the present invention's process, with no addition of lime or other chemical products. In fact, the filtered digestate obtained from the present invention's process is a liquid characterized by pH of 8.2-8.4 and a very significant buffer capacity determined by the included ionic species.

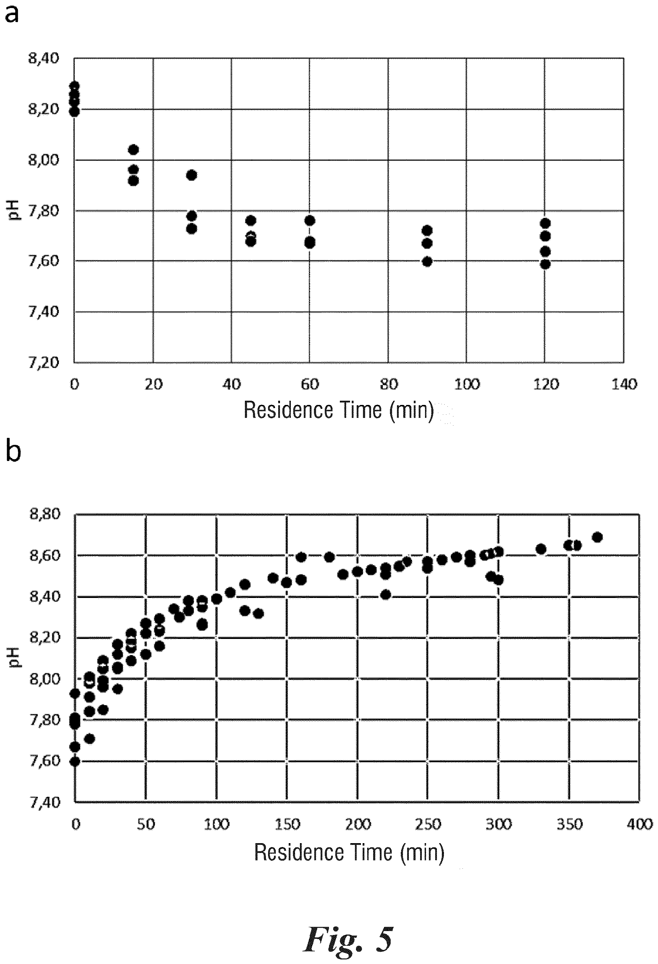

Moreover, this liquid has no settleable suspended solids and is therefore appropriate to be used as washing fluid for an acid gas like the biogas. The two main biogas pollutants (CO₂ and H₂S) are dissolved within the filtered digestate reducing its pH to 7.6-7.8 with 2 hours of residence time (Fig. 5a).

Preferably, in the invention's process the filtered digestate is further conveyed to a phase of stripping with air, for removal of inorganic nitrogen (NH₃).

The gaseous stripped effluent stream including ammonia is preferably conveyed to a scrubber, wherein it is washed in countercurrent with an acid aqueous solution, producing a nitrogenous stream containing commercial nitrogen salts.

Optionally, the final filtered digestate stream obtained after ammonia removal and the digestate extracted from anaerobic digestion are completely or partially mixed together, to be disposed on agricultural land.

Moreover, the present invention refers to a plant as described in claim 11 for the actuation of the process herein described.

Further characteristics and advantages of the present invention will be evident from the description of preferred but not exclusive embodiments of the invention, exemplified but not limited by attached drawings.

### BRIEF DESCRIPTION OF FIGURES

Figure 1: schematic view of a biogas production plant (100), according to a preferred embodiment of the present invention, comprising recirculation to the fermenter of the biomass and of part of the organic substrate.
Figure 2: schematic view of a biogas plant (200), according to a preferred embodiment, including the process portion shown Figure 1 and two additional sections, one for biogas purification and one for nitrogenous compounds recovery.
Figure 3: schematic view of a biogas plant (300), according to a preferred embodiment, including the process portion shown Figure 1 and two additional sections, one for nitrogenous compounds recovery and one for biogas purification.
Figure 4: schematic view of a portion of a biogas plant, according to a preferred embodiment of the present invention, comprising a section for thermal recovery from digestate and final filtrate by feed pre-heating.
Figure 5: a) graphic of pH variation of the filtered digestate kept at different residence times in countercurrent with biogas; b) graphic of pH variation of the filtered digestate kept at different residence times in the ammonia stripping tower. For each residence time in a) and b) the experiment has been replicated several times.

### DETAILED DESCRIPTION OF THE INVENTION

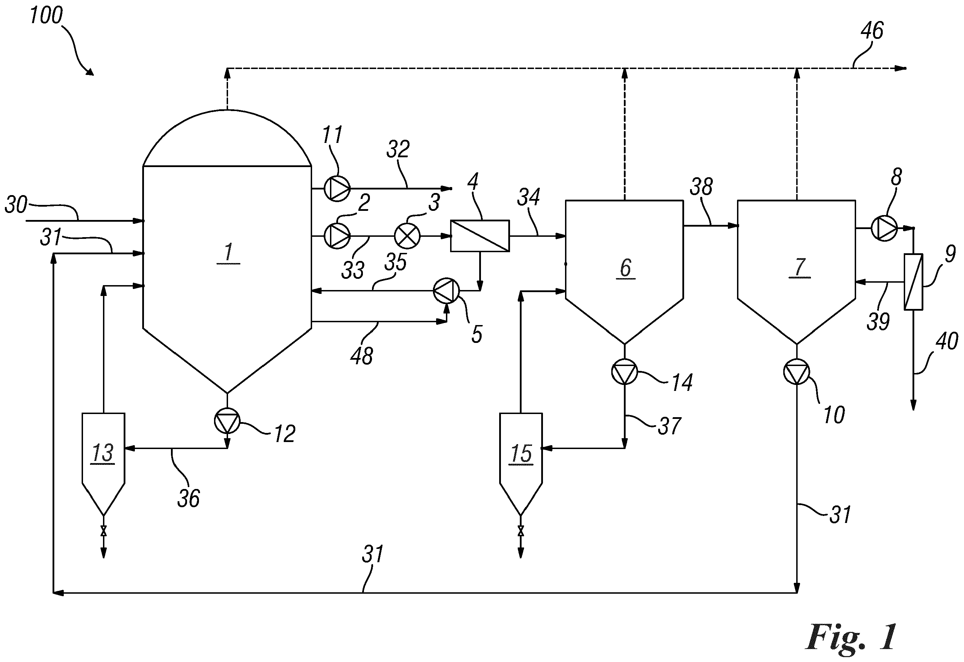

A preferred embodiment of the present invention is described below, with reference to plant schematic views showed in Figures 1-3.

An anaerobic fermenter (or digester, 1), thermoregulated and stirred, under anaerobic condition, is fed with a stream of organic material (organic substrate or organic matrix or feed, 30).

This organic material can include animal sewages, fibrous organic substrates or industrial or agricultural amylaceous organic materials; in particular it can include: by-products of the processing of cereals, fruit and vegetables and other similar substrates. Preferably this organic material comprises or consists in an organic waste material (organic waste), more preferably of zootechnical origin, most preferably swine or cattle sewages.

Preferably, said organic matrix is introduced into the fermenter without being previously treated (pre-treatment). Optionally, said organic matrix is pre-treated, for example by homogenization, flotation, or hydrolysis.

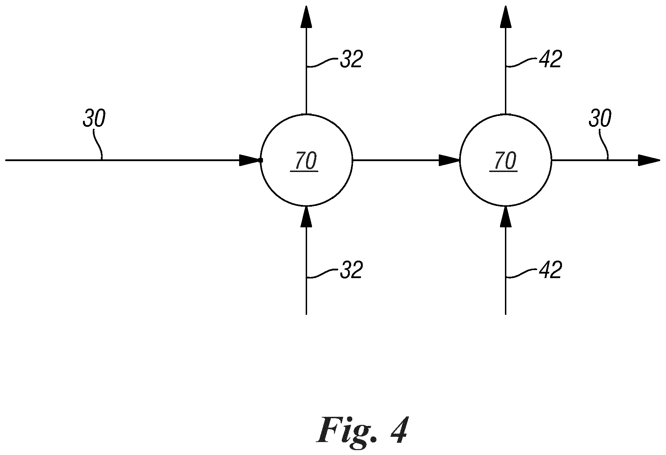

Preferably, said organic matrix is pre-heated by contact with the hot digestate outflowing from the digester (as shown in Figure 4).

The fermenter's (1) volume can vary depending on the nature of organic matrix fed. For example, if the organic matrix consists of sludges, the fermenter volume would require up to 20-40 days of hydraulic residence time (HRT), depending on the plant's degradation speed. If the feed mixture includes fibrous matrices as relevant component, the residence time will increase up to 40-60 days.

Part of the digestant is periodically extracted from digester (1) by means of a pump (2), through a digesting stream (33); preferably, it is conveyed to a shredder (3) that crushes the fibrous fraction making it then more easily attacked by bacteria. The digestant, extracted from fermenter (1), preferably passed through a shredder (3), is sent to a separator (4); said separator (4) is sealed, in order to minimize contact with air. This minimization of air contact is preferably achieved by connecting the drain for the separated solid with the recirculation pump (5), so that air exchange is limited. The solid fraction is reluctant to absorb oxygen, therefore this simple expedient, together with the minimum contact (below 10%) between liquid fraction and air, makes oxygen presence negligible during the separation phase.

The digesting stream (33) comprises at least a part of the digestant and at least a part of the bacterial biomass contained in the fermenter (1). Once in the separator (4) the digesting stream (33) is separated into: a digestate's liquid fraction (34), characterized by a percentage of dry matter below 6% by weight based on the total weight of the liquid fraction (w/w), preferably in the range of 1-4% w/w, and a denser fibrous fraction (35), characterized by dry matter content percentage above 15% by weight based on the total weight of the fibrous fraction (w/w), preferably between 20-35% w/w.

Preferably, said separator (4) is a mechanic solid-liquid separator. Preferably, the liquid stream of liquid digestate fraction (34) comprises particles with a diameter below 500 µm.

The fibrous stream (35) containing the digestant's fibrous fraction is recirculated to fermenter (1) under substantial anaerobic conditions, preferably by means of a recirculation pump (5). In this way the residence time of the organic matter's fibrous fraction is increased. Preferably the recirculation pump (5) is flanged to the separator (4) drain from which it receives the separate fibrous stream (35) in a hopper; a screw located inside the hopper pushes the solid fraction toward a dilution chamber where it meets a dilution liquid (48). Preferably the dilution liquid is the organic substrate (i.e. fresh sewage) feeding the plant (30); optionally, a part of the anaerobic fermentation digestant can be used. The screw presses the solid fraction into the dilution chamber minimizing contact between dilution liquid and air. This system, according to a preferred embodiment of the present invention, allows, with a considerable engineering plant simplicity, to recycle the fibrous matrix maintaining oxygen concentration in the recycling stream below 1 ppm.

The liquid stream (34), containing the greatest part of bacterial biomass of fermenter (1), is sent to a microfiltration unit.

According to the invention, the liquid stream (34) is previously sent to a first tank (7), upstream of the microfiltration unit; in addition, the liquid stream (34) is first sent to a further tank (6) placed between the microfiltration unit and the first tank (7), maintained at the same temperature of the fermenter (1) and under anaerobic condition.

A sand removal circuit is connected to said further tank (6). The sand removal circuit includes a sand removal (degritting) unit (15); preferably this sand removal unit is a dynamic settling vessel (sand thickener) fed with a continuous degritting stream (37), collected from the further tank (6), preferably through a pump (14); this degritting stream (37) directly returns into the further tank (6), while a sand discharge valve periodically drains ashes, sand and debris accumulating into the sand removal unit (15).

Periodically, preferably with calculated frequency, a stream comprising the liquid contained in the further tank (6) is transferred into the first tank (7).

Preferably, the first and further tanks (7, 6) are connected to the biogas line (46) deriving from the anaerobic digester (1); thus, the fermenter (1), by means of a gasometer, acts as a buffer for the volume changes occurring every time that a liquid transfer takes place from/to the first and further tanks (7, 6).

In an optional embodiment, the liquid stream (34), comprising the digestate liquid fraction coming from the separator (4), is directly conveyed to the first tank (7).

Said first tank (7) has the same temperature and anaerobic conditions of the fermenter (1). Preferably, said first tank (7) is connected to the microfiltration system (9); preferably, the microfiltration system comprises a circuit connected to the first tank (7) in which, through a pump (8), the stream outflowing from the first tank (7) is conveyed to a microfiltration membrane. Preferably said microfiltration system is a tangential microfiltration system.

A first stream of filtered digestate (40) substantially free of bacterial biomass flows out from the microfiltration system; on the other hand, a retained stream (39) in which the bacterial biomass is concentrated, is retained and returns into the system through the first tank (7): the liquid contained in the first tank (7), enriched with bacterial biomass after microfiltration, is periodically returned to the anaerobic fermenter (1) by a recirculating stream (31), preferably moved by a pump (10).

The process outlined above, according to a preferred embodiment of the invention, is therefore characterized by the recycle of the bacterial biomass to the fermenter, after microfiltration of the digestate liquid fraction, previously separated from the digestant's fibrous fraction by means of at least one separation stage; this digestate liquid fraction is also deprived of solid debris by means of passage in a tank.

The present invention plant comprises also the recycle to the fermenter of the organic matter fibrous fraction separated from the digestate by means of a separator; said fibrous fraction is conveyed to the fermenter by a recirculation pump (5) that minimizes contact with air, for example through a loading hopper for the fibrous fraction and a dilution chamber wherein contact with air is minimized.

The plant of the present invention can be adapted to various feed matrices by modulating the volume percentages of the streams of feed (30) and first filtered digestate (40).

For example, with a ratio of 50% (v/v) between the first filtered digestant (40) and the feed (30), the process outlined above allows to increase degradation efficiency of about 5%, compared to state of the art processes. Further increasing the first filtered digestant production, the process efficiency can increase up to maximum 8-9% compared to state of the art processes. That is, with the same plant power, feed can be reduced by a factor of 5-9% due to a greater degradation. Moreover, the ratio between the first filtered digestant stream (40) and the feed stream (30) determines the dry matter content in the anaerobic digester under stationary conditions, on the basis of a mass balance. Basically, the greater the volume of the clear fraction (of filtered digestate) produced daily, the higher the solids concentration in anaerobic digestion will be. Dry matter concentration must indicatively remain in a range of 3-10% by weight based on the total weight of digestant (w/w), preferably of 5-8% w/w; in fact, in case of extreme dilution, problems in maintaining process temperature would arise, while in case of extreme density there would be problems in fluids mixing and transferring.

Organic matter degradation produces ashes, rich in phosphorus. In traditional plants these ashes remain within the digesters producing sediments and precipitates that progressively reduce the available volume for fermentation. Preferably, in the present invention's process, a bottom stream is conveyed from the bottom of the fermenter (1), through a pump (12), into a sand thickener (13), preferably a dynamic thickener, that separates a part of heavier ashes discharging them through a valve, while a liquid part containing bacterial biomass and depurated from ashes is recirculated in the reactor (1).

The inorganic phosphorous included in the sediment of the sand removal unit (s) (13, 15) can be depurated and transformed into fine phosphorous salts by methods known in the art, or it can be directly disposed for agricultural use.

Preferably, the fermenter (1) comprises an overflow stream (32) comprising digestate, activated by a pump (11): the overflow stream purpose is to maintain a constant digestant level in the fermenter (1). Therefore, the present invention's plant preferably includes: one feed, represented by feed stream (30), and two outlets represented by the overflow and the first filtered digestate streams (32 and 40) .

The volumetric ratio between the first filtered digestate (40) and feed (30) streams depends on the recipe of the feed mixture, on the nitrogen removal requirements and on the level of purification required for the biogas. Indeed, the amount of the first filtered digestate that is produced daily shall be the lowest needed to achieve the minimum objectives of: bacterial biomass concentration, nitrogen removal, and biogas purification considering the costs/revenues optimization.

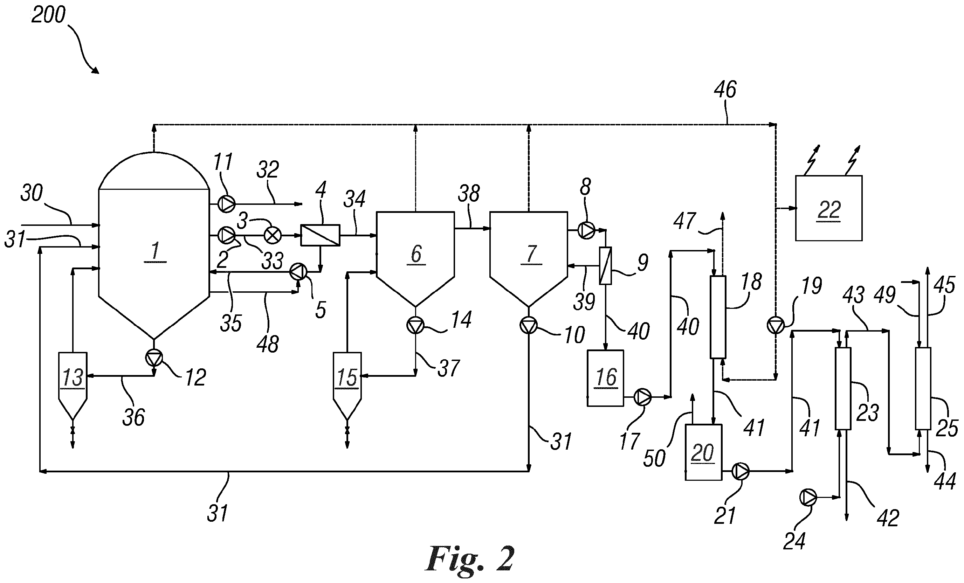

As shown in Figure 2, in a preferred embodiment, the digestate clear fraction (40) obtained after microfiltration can be used to wash the biogas produced by the fermenter, to reduce the concentration of H₂S, NH₃ and CO₂: the first stream of filtered digestate (40) is preferably collected in a tank (16) and conveyed by means of a pump (17) to a first scrubber (18) where it is put in counter-current contact with, all or part of, the biogas stream (46) produced by the fermenter (1) .

Through the contact between the biogas gaseous stream (46) and the first filtered digestate liquid stream (40), a transfer of CO₂ and H₂S from the gaseous phase to the liquid phase occurs. The first filtered digestate (40) is a liquid characterized by pH 8.2-8.4 and high buffer capacity (200-250 meq/L), therefore it is suitable to be used as washing fluid for biogas. Preferably, this first filtered digestate stream (40) has no suspended solids. Preferably, said first filtered digestate stream (40) represents the 50% in volume of the feed stream (30).

The purified biogas (47) coming out from said first scrubber (18) contains mostly CH₄ and in minor part H₂S, NH₃ and CO₂. If required, in a preferred aspect, purified biogas (47) flows to an upgrading phase that removes the remaining pollutants allowing biogas classification to "biomethane". It is noted that this finishing phase of biogas to biomethane occurs in conditions that are favored by the upstream process which has already removed a very significant part of pollutants without chemicals addition and which minimizes subsequent upgrading costs. The greater the first filtered digestate production, the higher the purity level obtained for purified biogas (47).

In a preferred embodiment, biogas' pollutants scrubbing process takes place at a pressure of 5-7 bar.g by pressurization with a compressor (19). The liquid flowing out from the first scrubber (18) is collected in a tank (20, flash tank) where it is returned to atmospheric pressure by releasing vents rich in CO₂ (50). Optionally, these vents can be exploited by CO₂ recover as by-product of the process, according to known techniques.

After being used as fluid for biogas scrubbing, a second stream of filtered digestate (41) is obtained, characterized by pH of 7.6-7.8 and a concentration of ammoniacal nitrogen substantially unchanged compared to the first filtered digestate stream (40).

The degradation of the organic matter occurring during the anaerobic digestion converts nitrogen and phosphorus into inorganic form, to the detriment of the organic form; the greater the organic matter degradation, the higher the transformation from organic to inorganic form of nitrogen and phosphorus. It is noted that the prevailing nitrogen inorganic form passes together with the first filtered digestate stream (40) into the second filtered digestate stream (41) after biogas scrubbing.

In a preferred embodiment, the second clear filtered digestate (41) obtained after biogas scrubbing is conveyed to a stripping tower (23) for ammonia nitrogen removal.

The stripping tower (23) is a properly sized and thermoregulated vessel in which air is blown from the bottom by a compressor (24). The thermal regulation of the process at 60-70°C shifts the ammonia dissociation equilibrium toward the gaseous phase (NH₃), promoting removal of the same. Blown air facilitates extraction from the filtered digestate of ammonia, which flows through vents, included in a gaseous stream (43). Air insufflation also determines gaseous CO₂ extraction which causes a gradual increase in pH that rises together with the increase of the residence time. However, since CO₂ extraction occurs simultaneously with the NH₃ removal, there is a reduction of the overall filtrate alkalinity (of about 50% with 5 hours residence time). In particular, the filtered digestate liquid outflowing from the stripping tower is characterized by a NH₃ concentration reduced by 65-70% compared to the liquid entering the stripping tower, and by a pH of about 8.4-8.8, with however an alkalinity reduced of about 40-60% (Figure 5b).

Optionally, the final filtered digestate stream obtained after ammonia removal (stream 42) and the digestate extracted from anaerobic digestion (the overflow stream 32) are completely or partially mixed together, to be disposed on agricultural land.

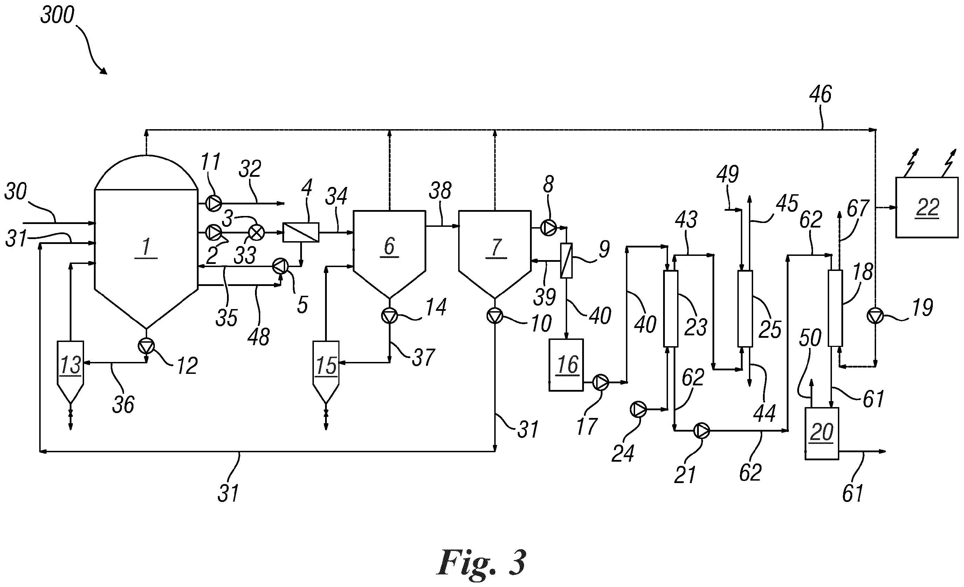

Optionally, the ammonia stripping phase directly occurs downstream of the microfiltration and biogas is subsequently washed with the liquid resulting from stripping. This can be useful if the flow rate of the biogas to be purified is negligible or if purification is not required at all. Indeed, in this second configuration (shown in Figure 3), efficiency of ammoniacal nitrogen removal would remain unchanged, but the filtrated digestate's capacity to adsorb biogas CO₂ would be reduced of about 50%. Therefore, in this second configuration the first filtered digestate stream (40), preferably collected in a tank (16), first flows to the stripping tower (23) and then the effluent digestate stream (62), with a reduced concentration of nitrogen, flows to the first scrubber (18) for biogas purification, as described above; from said first scrubber (18) thus exits a gaseous stream of purified biogas (67) and an outward third stream of digestate (61) that has a lower pH compared to the effluent digestate stream (62).

Preferably, ammonia extracted in the stripping tower (23) can subsequently be captured in the form of nitrogen salts by passing through a second scrubber (25), where it is put in counter-current contact with an acid solution (49) allowing ammonia transfer into the aqueous phase. Preferably this acid solution (49) consists of a sulfuric acid solution (H₂SO₄). With this step, an ammonium sulfate (NH₄)₂SO₄ solution is obtained, exiting from the second scrubber (25) as nitrogenous stream (44), that can be used to produce inorganic fertilizers; vents (45) also exit from the second scrubber (25).

Optionally, downstream of the phase of removal of nitrogenous compounds from the second stream of filtered digestate (41), a phase of nitrogenous salt concentration is furtherly provided by an evaporation/crystallization step.

Preferably the present invention's process also comprises a phase of heat recovery from the digestate by heat exchange with the feed (30). In particular, the overflow digestate (32) and the first filtered digestate (40) outflowing from the plant, being at 35-45°C and 55-70°C respectively, are preferably used to pre-heat the plant feed by passing through a shell and tube heat exchanger (70), as showed in Figure 4. Thus, it is possible to feed even very diluted matrices without making the system thermal balance unsustainable, requiring external heat sources in addition to the electrical cogeneration unit and heat recovery from digestate.

In a preferred configuration a thermal engine (22) is included in the plant of the invention. This thermal engine (22) is fed with a part of the biogas produced in the present invention's process, upstream of the biogas depuration step. Preferably this thermal engine (22) produces electrical power partially destined for the plant's needs and partially sold to the national network. This thermal engine (22) also produces the heat necessary for the plant's heating requirements.

Preferably, the plant of the present invention is automatized by a suitable software.

The process of the present invention has greater degradation efficiency than traditional processes, thus producing a greater amount of biogas, with greater efficiency. Moreover, this process allows to mineralize a greater amount of organic load. In fact, the greater degradation of the introduced organic loads makes the fertilizer elements (nitrogen and phosphorus) more usable and assimilable by plants, as a consequence of the greater presence of NH₄ and PO₄ ions in the digestate.

The process of the present invention allows to increase the production capacity of a biogas production plant, overcoming the constraint of the cogeneration unit size. This makes the present invention's process advantageously applicable also to existing plants without generating problems related to the inevitable increase in plant's energy self-consumption. The energy surplus that the plant can produce can be in fact sold outside in the form of biogas, preferably purified (or as biomethane), generating a source of additional income covering the costs for nutrients recovery.

### EXAMPLES

Results obtained combining laboratory experiments and process numerical simulation are reported as examples. In particular, degradation efficiency data of various biogas production plants have been elaborated, measuring the gas yield potential of the effluent digestate. Laboratory scale tests have been conducted for biogas purification with the filtrated digestate produced by a commercial test apparatus. These tests were conducted at atmospheric pressure and obtained results were used as starting data for numerical simulations at higher pressures. Tests were conducted of nitrogen stripping from the outlet filtrate from the upgrading phase and total nitrogen and ammonia nitrogen values were sampled in all phases.

Alkalinity tests of outlet products from the various phases of the process have been carried out in order to support numerical simulations of chemical equilibrium. The analytical set thus obtained was used to set up a mass balance of main components within the various streams of the plant.

### EXAMPLE 1

In a first example, a more diluted feed, consisting of animal sewages only, is conveyed to the fermenter of a plant (200), according to a preferred embodiment of the present invention, and a stream volume ratio of first filtered digestate stream (40) to feed stream (30) is set at 60%; results for this example are displayed in Table 1.

It is noted that in this case the dry substance content exiting from the fermenter (1) is of about 6.7%.

**Table 1**

| **STREAMS** | | **30** | **33** | **34** | **35** | **40** | **31** | **32** | **42** | **After 13; 15** |
|---|---|---|---|---|---|---|---|---|---|---|
| Flow rate | m³/d | 710 | 757 | 632 | 405 | 426 | 203 | 282 | 426 | 2,5 |
| Temperatu re | °C | 20 | 42 | 42 | 42 | 42 | 42 | 42 | 60 | 42 |
| pH | - | 7,4 | 7,8 | 7,8 | 7,8 | 7,9 | 7,8 | 7,8 | 8,7 | 7,6 |
| DRY MATTER | % (w/w) | 5,8 | 6,7 | 2,5 | 11 | 1,3 | 5 | 7 | 1,3 | 3,8 |
| VOLATILE DRY MATTER | % (w/w) | 4,1 | 5 | 1,9 | 8,3 | 0,6 | 2,3 | 5,3 | 0, 6 | 1,3 |
| COD (CHEMICAL OXYGEN DEMAND) | g/L | 67,9 | 72 | 47,5 | 47,5 | 5,5 | 115 | 47,5 | 3 | 52 |
| N-tot | g/L | 4,72 | 4,95 | 4,72 | 4,72 | 4 | 4,6 | 4,72 | 1,4 | 5 |
| N-NH3 | g/L | 1,83 | 2,12 | 2, 6 | 2,6 | 2,8 | 2,32 | 2,6 | 0, 98 | 2 |
| P-tot | g/L | 1,57 | 3,03 | 2,8 | 2, 8 | 0,02 | 6, 96 | 2,8 | 0,02 | 6 |
| P-P04 | g/L | 0,86 | 1,87 | 1,8 | 1,8 | 0,02 | 4,6 | 1,8 | 0,02 | 1 |

### EXAMPLE 2

In a second example, a feed consisting of animal sewages and by-products is conveyed to the fermenter of a plant (200), according to a preferred embodiment of the present invention, and a stream volume ratio of first filtered digestate stream (40) to feed stream (30) is set at 30%; results for this example are displayed in Table 2.

It is noted that in this case the dry substance content exiting the fermenter (1) is of about 8.2%.

**Table 2**

| **Stream** | | **30** | **33** | **34** | **35** | **40** | **31** | **32** | **42** | **After 13;15** |
|---|---|---|---|---|---|---|---|---|---|---|
| Flow rate | m³/d | #### | 185,0 | 159,0 | 81,0 | 107,0 | 50,0 | 247,0 | 107,0 | 2,5 |
| Temperatu re | °C | 20,0 | 42,0 | 42,0 | 42,0 | 42,0 | 42,0 | 42,0 | 60,0 | 42,0 |
| pH | - | 7,4 | 7,8 | 7,8 | 7,8 | 7,9 | 7,8 | 7,8 | 8,7 | 7,6 |
| DRY MATTER | % (w/w) | 0,1 | 0,1 | 0,0 | 0,2 | 0,0 | 0,1 | 0,1 | 0,0 | 0,0 |
| VOLATILE DRY MATTER | % (w/w) | 0,1 | 0,1 | 0,0 | 0,1 | 0,0 | 0,0 | 0,0 | 0,0 | 0,0 |
| COD (CHEMICAL OXYGEN DEMAND) | g/L | 68,8 | 72,6 | 48,2 | 48,2 | 5,5 | 115,6 | 48,2 | 3,0 | 52,0 |
| N-tot | g/L | 3, 93 | 3,91 | 3,93 | 3,93 | 4,00 | 4,60 | 3,93 | 1,40 | 5,00 |
| N-NH3 | g/L | 1,38 | 1,66 | 2,60 | 2,60 | 2,80 | 2,32 | 2,60 | 0,98 | 2,00 |
| P-tot | g/L | 0, 98 | 2,06 | 2,80 | 2,80 | 0,02 | 6,96 | 2,80 | 0,02 | 6,00 |
| P-PO4 | g/L | 0,49 | 1,24 | 1,80 | 1,80 | 0,02 | 4,60 | 1,80 | 0,02 | 1,00 |

Through appropriate laboratory analysis, it is also possible to quantify not only the dry matter in the reactor as control parameter, but also the methanogen biomass density.

As for the solids concentration, also the bacterial biomass is even more concentrated as the filtrate daily production increases compared to the feed.

### EXAMPLE 3

CO₂ concentration after biogas purification according to a preferred method of the present invention (as with the plant shown in Figure 2) was evaluated. CO₂ concentration decreases by at least 50% with a minimum amount of 50-100 liters of filtrate per kg of total CO₂.

### EXAMPLE 4

The energetic efficiency of a plant according to the invention, such as the one shown in figure 1, compared to a plant according to prior art (such as the one disclosed in US2009206028) has been evaluated.

The results are shown in Table 3.

**Table 3**

| | | | Comparative plant | Invention plant | Comparative plant | Invention plant |
|---|---|---|---|---|---|---|
| | | | diluted feed | | dense feed | |
| Feed | swine sewage | ton/day | 207 | 207 | 76 | 76 |
| | Cattle sewage | ton/day | 0 | 0 | 50 | 50 |
| | Wheat silage | ton/day | 10 | 8,9 | 10 | 7,7 |
| | Microfiltration rate | % of feed | 0 | 60% | 0 | 30% |
| | Generated power | kW | | 299 | | |

In both the cases shown in table 3, one with a more diluted recipe and one with a denser recipe, the recirculation of fibers and bacterial biomass according to the invention's process allows to produce the same quantity of biogas produced by a process of the prior art, but with less feed: 215.9 tons instead of 217 in the first case, 133.7 tons against 136 in the second case. In particular, it is noted that the process of the invention can generate the same power of a process of the prior art, using less wheat silage (that is the organic feed with the highest energy content). The sewages amounts have been kept deliberately equal in all the cases. Therefore, in terms of dry substance, the process of the invention has a feed/energy benefit of about 9.5% in the first case and 5% in the second case, compared to a prior art process.

### EXAMPLE 5

The biogas washing efficiency of a plant according to a preferred embodiment of the invention, such as the one shown in figure 2, compared to that of a plant according to the prior art (such as the one disclosed in US2009206028) has been evaluated.

**Table 4**

| | | | Comparative plant | Invention plant |
|---|---|---|---|---|
| | | | diluted feed | |
| Feed | swine sewage | ton/day | 207 | 207 |
| | cattle sewage | ton/day | 0 | 0 |
| | wheat silage | ton/day | 10 | 8,9 |
| | microfiltration rate | % of feed | 0 | 60% |
| | filtered liquid* | ton/day | ≈200 | ≈125 |
| | filtered liquid ** Alkalinity | meq/L | ≈90 | ≈250 |
| | biogas/liquid ratio (v/v) | - | 9,6 | 28,8 |
| | Biogas CO2 initial | % | 34,50% | 45% |
| | Biogas CO2 final | % | 15% | 22% |
| | CO2 reduction | % | 43% | 49% |
| | generated power | kW | **299** | |

| | | | | |
|---|---|---|---|---|
| * for the comparative plant the filtered liquid is the effluent stream after the pH adjustment with lime and after the ammonia stripping phase; for the plant of the invention this is the first filtered stream (34) ** for the comparative plant this is the alkalinity of the liquid without considering the lime addition. After pH adjustment the alkalinity is increased in order to wash the biogas | | | | |

The results are shown in Table 4.

Equal or even greater reduction of the CO₂ of the biogas (i.e. a more efficient washing of the biogas) is obtained by a process according to the invention, compared to a process of the prior art, thanks to a more efficient process that does not require pH adjustment of the biogas counter-current washing stream with chemical additives, such as lime.

In the prior art process, the step of ammonia stripping reduces the alkalinity of the digestate liquid, consequently lime must be dosed in order to restore a high pH before washing the biogas; in a process according to a preferred embodiment the invention, the filtered digestate is directly used to wash the biogas, without the need of adjusting its pH.

## Claims

1. A process for the production of biogas, comprising:
i) a phase of anaerobic digestion of an organic substrate (30) in a fermenter (1), by means of bacterial biomass, forming a digesting mass in said fermenter (1), wherein from said fermenter (1) flow out: a biogas stream (46) consisting of biogas, and at least one digesting stream (33) comprising at least a fraction of the digesting mass;
ii) a phase of separation of the digesting stream (33), wherein said digesting stream (33) is conveyed to a separator (4), that separates a fibrous stream (35), comprising the fibrous fraction of the digesting stream, and a liquid stream of liquid digestate (34) comprising the liquid fraction of the digesting stream and the bacterial biomass, and wherein said separator is sealed in order to minimize air contact;
iii) a phase of recirculation of the fibrous stream (35) to the fermenter (1), wherein said fibrous stream (35) is substantially free from oxygen;
iv) a phase of concentration of the bacterial biomass contained in the liquid stream (34) by microfiltration, wherein: said liquid stream (34) is conveyed periodically to at least a first tank (7) maintained under the same anaerobic conditions and temperature of the fermenter (1) from a further tank (6), that is placed between the separator (4) and the first tank (7) and is connected to a degritting circuit comprising a sand removal unit (15), wherein a degritting stream (37) is withdrawn continuously from the further tank (6), conveyed to the degritting circuit for removal of the ashes, and then sent back to the further tank (6), removed of ashes; said first tank (7) being connected to a microfiltration system (9) comprising a microfiltration membrane, through which part of the liquid stream (34) is microfiltered obtaining a first stream of filtered digestate (40) substantially free from bacterial biomass, and part of the liquid stream (34) is retained, obtaining a retained stream (39) comprising the concentrated bacterial biomass that re-enters into the first tank (7);
v) a phase of recirculation of the bacterial biomass to the fermenter (1), wherein a recirculating stream (31) comprising the bacterial biomass flows out of the first tank (7) and is recirculated to the fermenter (1).

2. A process according to claim 1, further comprising a phase vi) of biogas purification, wherein the first stream of filtered digestate (40) exiting the microfiltration system (9) is conveyed to a first scrubber (18) for biogas washing, within which it is put in counter-flow with at least a part of the biogas stream (46) that is produced by the fermenter (1); wherein from said first scrubber (18) exits a gaseous stream of purified biogas (47) and an outward second stream of filtered digestate (41) that has a lower pH than the inward first stream of filtered digestate (40) .

3. A process according to claim 2 further comprising a phase vii) of removal of nitric compounds from the digestate, wherein the second stream of filtered digestate (41) exiting the first scrubber for biogas washing (18) is conveyed to a stripping tower (23) for the removal of inorganic nitric compounds, obtaining a final stream of filtered digestate (42) that exits the stripping tower (23) with a reduced nitrogen content, relative to the incoming second stream of filtered digestate (41), and a gaseous stream comprising ammonia (43), that flows out of vents of the stripping tower (23).

4. The process of claim 3, wherein the gaseous stream comprising ammonia (43), flowing out of vents of the stripping tower's (23), is conveyed to a second scrubber (25) and contacted in counter-flow with an acidic solution (49), obtaining a nitrogenous stream (44) comprising nitrogen salts, exiting the second scrubber (25).

5. A process according to claim 1 further comprising a phase vi-b) of removal of nitric compounds from the digestate, wherein the first stream of filtered digestate (40) exiting the microfiltration system (9) is conveyed to a stripping tower (23) for the removal of inorganic nitric compounds, obtaining an effluent digestate stream (62), that exits the stripping tower (23) with a reduced nitrogen content relative to the incoming first stream of filtered digestate (40), and a gaseous stream comprising ammonia (43) .

6. The process of claim 5, further comprising a phase vii-b) of biogas purification, wherein the effluent digestate stream (62) that exits the stripping tower (23) is conveyed to a first scrubber for biogas washing (18), within which it is put in counter-flow with at least a part of the biogas stream (46) that is produced by the fermenter (1), wherein from said first scrubber (18) exits a gaseous stream of purified biogas (67) and an outward third stream of digestate (61) that has a lower pH compared to the effluent digestate stream (62).

7. A process according to any one of claims 5 or 6, wherein the gaseous stream comprising ammonia (43), flowing out of vents of the stripping tower's (23), is conveyed to a second scrubber (25) and contacted in counter-flow with an acidic solution (49), obtaining a nitrogenous stream (44) comprising nitrogen salts, exiting the second scrubber (25) .

8. A process according to any one of the preceding claims comprising a phase viii) wherein a bottom stream (36) exits from the bottom of the fermenter (1) and is conveyed to a sand thickener (13), within which a first portion of the bottom stream (36) comprising phosphorus-rich ashes, and a second liquid portion, containing the bacterial biomass purified from ashes, are separated, and wherein said second liquid portion is reintroduced into the fermenter (1).

9. A process according to any one of the preceding claims, wherein in phase iii) said fibrous stream (35) comprising the fibrous fraction of the digesting mass is recirculated to the fermenter (1) by means of a recirculation pump (5), wherein the inside of said recirculation pump (5) is in conditions of substantial anaerobiosis, wherein said recirculation pump (5) comprises a loading hopper that receives the fibrous stream (35) from the separator, and a dilution chamber comprising a dilution liquid (48), and wherein the fibrous stream (35) is conveyed from the loading hopper to the dilution chamber and from it to the fermenter (1).

10. A process according to any one of the preceding claims comprising a phase ix) in which an overflow stream (32) comprising part of the digesting mass is withdrawn from the fermenter (1), and wherein the volume of organic matter within the fermenter (1) is maintained constant in time by adjusting the proportion of the overflow stream (32) to the digesting stream (33) outgoing the fermenter (1).

11. A biogas production plant, comprising:
a) at least one anaerobic fermenter (1) for biogas production, which is fed with a feed stream (30) comprising organic substrate and bacterial biomass, and from which at least a digesting stream (33), comprising at least a fraction of a digesting mass, exits;
b) a separator (4) connected to said fermenter (1) and receiving the digesting stream (33) that exits the fermenter (1), said separator (4) being capable of separating at least a liquid stream (34) comprising a liquid fraction of digestate and at least part of the bacterial biomass, and a fibrous stream (35), comprising a fibrous fraction of the digesting product;
c) a microfiltration system (9) for concentrating the bacterial biomass contained in the liquid stream (34), comprising a microfilter membrane through which the liquid stream (34) is filtered, thus obtaining a first stream of filtered digestate (40), substantially free of bacterial biomass, and a retained stream (39) comprising concentrated bacterial biomass;
d) a first tank (7) placed between the separator (4) and the microfiltration system (9), into which the retained stream (39) comprising concentrated bacterial biomass is sent, and from which the bacterial biomass is periodically recirculated to the anaerobic fermenter (1) through a recirculating stream (31); wherein said first tank (7) is kept at the same temperature of the fermenter (1) in anaerobiosis conditions and it is connected to a biogas line (46) coming from the fermenter (1);
e) a further tank (6) placed downstream of the separator (4) and upstream the first tank (7), said further tank (6) being maintained at the same temperature as the fermenter (1) and under anaerobic conditions, wherein said further tank (6) receives the liquid stream (34) from the separator (4), and wherein said further tank (6) is connected to a degritting circuit comprising a sand removal unit (15), said sand removal unit (15) comprising a valve for ashes discharge, so that a liquid freed from ashes is periodically poured from the further tank (6) into the first tank (7).

12. The plant of claim 11, further comprising:
f) a shredder (3) placed between said fermenter (1) and said separator (4).

13. The plant of claims 11 or 12, further comprising at least one of:
g) a first scrubber (18) for biogas washing, within which at least a portion of the biogas (46) produced in the fermenter (1) is washed in counter-flow with a stream of digestate (40, 62);
h) a stripping tower (23) for the removal of organic nitric compounds from a stream of filtered digestate (40, 41), capable of providing a final stream of filtered digestate (42) having a reduced content of nitrogen compounds; preferably said stripping tower being connected to a second scrubber (25) for the mineralization of organic nitrogen compounds that are present in the gaseous stream comprising ammonia (43) that exits from the stripping tower (23), and wherein said gaseous stream (43) is put in counter-flow with an acidic solution (49).

14. The plant of claim 13, comprising
h) the stripping tower (23) for the removal of organic nitric compounds from a stream of filtered digestate (40, 41), capable of providing a final stream of filtered digestate (42) having a reduced content of nitrogen compounds; and
i) a section for heat exchange by means of a tube bundle heat exchanger, wherein the feed stream (30) comprising organic substrate and bacterial biomass is put in contact with an overflow stream (32) exiting the fermenter (1) and with the final stream of filtered digestate (42) having a reduced content of nitrogen compounds that exits from the stripping tower (23).

## Patentansprüche

1. Verfahren zur Herstellung von Biogas, umfassend:
i) eine Phase anaerober Vergärung eines organischen Substrats (30) in einem Fermenter (1) mittels bakterieller Biomasse, die eine Gärungsmasse in dem Fermenter (1) bildet, wobei Folgendes aus dem Fermenter (1) ausfließt: ein aus Biogas bestehender Biogasstrom (46) und mindestens einen Gärungsstrom (33), der mindestens einen Teil der Gärungsmasse umfasst;
ii) eine Trennungsphase des Gärungsstroms (33), wobei der Gärungsstrom (33) zu einem Abscheider (4) geleitet wird, der einen faserigen Strom (35), umfassend die faserige Fraktion des Gärungsstroms, und einen flüssigen Strom aus flüssigem Gärrest (34), umfassend die flüssige Fraktion des Gärungsstroms und die bakterielle Biomasse, trennt, und wobei der Abscheider abgedichtet ist, um einen Luftkontakt zu minimieren;
iii) eine Rückführungsphase des faserigen Stroms (35) zu dem Fermenter (1), wobei der faserige Strom (35) im Wesentlichen frei von Sauerstoff ist;
iv) eine Konzentrationsphase der in dem flüssigen Strom (34) enthaltenen bakteriellen Biomasse durch Mikrofiltration, wobei: der flüssige Strom (34) von einem weiteren Tank (6), der zwischen dem Abscheider (4) und dem ersten Tank (7) angeordnet und mit einem Entkörnungskreislauf umfassend eine Sandentfernungseinheit (15) verbunden ist, periodisch zu mindestens einem ersten Tank (7) geleitet wird, der unter denselben anaeroben Bedingungen und derselben Temperatur des Fermenters (1) gehalten wird, wobei ein Entkörnungsstrom (37) kontinuierlich aus dem weiteren Behälter (6) entnommen, zur Entfernung der Asche in den Entkörnungskreislauf geleitet und dann von der Asche befreit in den weiteren Behälter (6) zurückgeführt wird; wobei der erste Tank (7) mit einem Mikrofiltrationssystem (9) verbunden ist, umfassend eine Mikrofiltrationsmembran, durch die ein Teil des flüssigen Stroms (34) mikrofiltriert wird, wodurch ein erster Strom von gefiltertem Gärrest (40) erlangt wird, der im Wesentlichen frei von bakterieller Biomasse ist, und ein Teil des flüssigen Stroms (34) zurückgehalten wird, wodurch ein zurückgehaltener Strom (39) erlangt wird, der die konzentrierte bakterielle Biomasse umfasst, die wieder in den ersten Tank (7) eintritt;
v) eine Rückführungsphase der bakteriellen Biomasse zu dem Fermenter (1), wobei ein Rückführungsstrom (31) umfassend die bakterielle Biomasse aus dem ersten Tank (7) ausströmt und zu dem Fermenter (1) zurückgeführt wird.

2. Verfahren nach Anspruch 1, ferner umfassend eine Phase vi) einer Biogasaufreinigung, wobei der erste Strom von gefiltertem Gärrest (40), der aus dem Mikrofiltrationssystem (9) austritt, zu einem ersten Wäscher (18) zum Waschen von Biogas geleitet wird, worin er in Gegenstrom mit mindestens einem Teil des Biogasstroms (46) gebracht wird, der von dem Fermenter (1) erzeugt wird; wobei aus dem ersten Wäscher (18) ein gasförmiger Strom von aufgereinigtem Biogas (47) und ein äußerer zweiter Strom von gefiltertem Gärrest (41), der einen niedrigeren pH-Wert als der innere erste Strom von gefiltertem Gärrest (40) aufweist, austritt.

3. Verfahren nach Anspruch 2, ferner umfassend eine Phase vii) einer Entfernung von Stickstoffverbindungen aus dem Gärrest, wobei der zweite Strom von gefiltertem Gärrest (41), der aus dem ersten Wäscher zum Waschen von Biogas (18) austritt, zu einem Abscheiderturm (23) zur Entfernung von anorganischen Stickstoffverbindungen geleitet wird, wodurch ein abschließender Strom von gefiltertem Gärrest (42), der aus dem Abscheiderturm (23) mit einem reduzierten Stickstoffgehalt in Bezug auf den eingeleiteten zweiten Strom von gefiltertem Gärrest (41) austritt, und ein gasförmiger Strom umfassend Ammoniak (43), der aus den Entlüftungsöffnungen des Abscheiderturms (23) strömt, erlangt wird.

4. Verfahren nach Anspruch 3, wobei der gasförmige Strom umfassend Ammoniak (43), der aus den Entlüftungsöffnungen des Abscheiderturms (23) ausströmt, zu einem zweiten Wäscher (25) geleitet wird und in Gegenstrom mit einer sauren Lösung (49) in Kontakt gebracht wird, wodurch ein stickstoffhaltiger Strom (44) umfassend Stickstoffsalze erlangt wird, der aus dem zweiten Wäscher (25) austritt.

5. Verfahren nach Anspruch 1, ferner umfassend eine Phase vi-b) einer Entfernung von Stickstoffverbindungen aus dem Gärrest, wobei der erste Strom von gefiltertem Gärrest (40), der aus dem Mikrofiltrationssystem (9) austritt, zur Entfernung von anorganischen Stickstoffverbindungen zu einem Abscheiderturm (23) geleitet wird, wodurch ein ausfließender Gärreststrom (62), der mit einem reduzierten Stickstoffgehalt in Bezug auf den eingeleiteten ersten Strom von gefiltertem Gärrest (40) aus dem Abscheiderturm (23) austritt, und ein gasförmiger Strom umfassend Ammoniak (43) erlangt wird.

6. Verfahren nach Anspruch 5, ferner umfassend eine Phase vii-b) einer Biogasaufreinigung, wobei der ausfließende Gärreststrom (62), der aus dem Abscheiderturm (23) austritt, zu einem ersten Wäscher zum Waschen von Biogas (18) geleitet wird, worin er in Gegenstrom mit mindestens einem Teil des Biogasstroms (46) gebracht wird, der von dem Fermenter (1) erzeugt wird, wobei aus dem ersten Wäscher (18) ein gasförmiger Strom von aufgereinigtem Biogas (67) und ein äußerer dritter Gärreststrom (61), der einen niedrigeren pH-Wert im Vergleich zu dem ausfließenden Gärreststrom (62) aufweist, austritt.

7. Verfahren nach einem der Ansprüche 5 oder 6, wobei der Gasstrom umfassend Ammoniak (43), der aus den Entlüftungsöffnungen des Abscheiderturms (23) ausströmt, zu einem zweiten Wäscher (25) geleitet und in Gegenstrom mit einer sauren Lösung (49) in Kontakt gebracht wird, wodurch ein stickstoffhaltiger Strom (44) umfassend Stickstoffsalze erlangt wird, der aus dem zweiten Wäscher (25) austritt.

8. Verfahren nach einem der vorherigen Ansprüche, umfassend eine Phase viii), wobei ein Bodenstrom (36) aus dem Boden des Fermenters (1) austritt und zu einem Sandverdicker (13) geleitet wird, worin ein erster Teil des Bodenstroms (36) umfassend phosphorreiche Asche und ein zweiter flüssiger Teil, der die von der Asche aufgereinigte bakterielle Biomasse enthält, abgeschieden werden, und wobei der zweite flüssige Teil wieder in den Fermenter (1) eingeführt wird.

9. Verfahren nach einem der vorherigen Ansprüche, wobei in Phase iii) der faserige Strom (35) umfassend die faserige Fraktion der Gärungsmasse mittels einer Umwälzpumpe (5) zu dem Fermenter (1) zurückgeführt wird, wobei die Innenseite der Umwälzpumpe (5) sich unter Bedingungen einer wesentlichen Anaerobiose befindet, wobei die Umwälzpumpe (5) einen Beschickungstrichter, der den faserigen Strom (35) von dem Abscheider erhält, und eine Verdünnungskammer umfassend eine Verdünnungsflüssigkeit (48) umfasst, und wobei der faserige Strom (35) von dem Beschickungstrichter zu der Verdünnungskammer und von dort zu dem Fermenter (1) geleitet wird.

10. Verfahren nach einem der vorherigen Ansprüche, umfassend eine Phase ix), in der ein Überlaufstrom (32) umfassend einen Teil der Gärungsmasse aus dem Fermenter (1) entnommen wird, und wobei das Volumen der organischen Substanz innerhalb des Fermenters (1) zeitlich konstant gehalten wird, indem das Verhältnis des Überlaufstroms (32) zu dem aus dem Fermenter (1) austretenden Gärungsstrom (33) angepasst wird.

11. Anlage zur Biogaserzeugung, umfassend:
a) mindestens einen anaeroben Fermenter (1) zur Biogaserzeugung, der mit einem Zuführstrom (30) umfassend organisches Substrat und bakterielle Biomasse beschickt wird und aus dem mindestens ein Gärungsstrom (33) umfassend mindestens eine Fraktion einer Gärungsmasse austritt;
b) einen Abscheider (4), der mit dem Fermenter (1) verbunden ist und den aus dem Fermenter (1) austretenden Gärungsstrom (33) erhält, wobei der Abscheider (4) in der Lage ist, mindestens einen flüssigen Strom (34) umfassend eine flüssige Fraktion des Gärrests und mindestens einen Teil der bakteriellen Biomasse, und einen faserigen Strom (35) umfassend eine faserige Fraktion des Gärungsprodukts abzuscheiden;
c) ein Mikrofiltrationssystem (9) zum Konzentrieren der bakteriellen Biomasse, die in dem flüssige Strom (34) enthalten ist, umfassend eine Mikrofiltermembran, durch die der flüssige Strom (34) gefiltert wird, wodurch ein erster Strom von gefiltertem Gärrest (40), der im Wesentlichen frei von bakterieller Biomasse ist, und ein zurückgehaltener Strom (39) umfassend konzentrierte bakterielle Biomasse erlangt wird;
d) einen ersten Tank (7), der zwischen dem Abscheider (4) und dem Mikrofiltrationssystem (9) angeordnet ist, in den der zurückgehaltene Strom (39) umfassend konzentrierte bakterielle Biomasse geleitet wird und aus dem die bakterielle Biomasse periodisch durch einen Rückführungsstrom (31) zu dem anaeroben Fermenter (1) zurückgeführt wird; wobei der erste Tank (7) unter Anaerobiosebedingungen auf der gleichen Temperatur wie der Fermenter (1) gehalten wird und mit einer Biogasleitung (46) verbunden ist, die von dem Fermenter (1) kommt;
e) einen weiteren Tank (6), der stromabwärts von dem Abscheider (4) und stromaufwärts von dem ersten Tank (7) angeordnet ist, wobei der weitere Tank (6) auf der gleichen Temperatur wie der Fermenter (1) und unter anaeroben Bedingungen gehalten wird, wobei der weitere Tank (6) den flüssigen Strom (34) von dem Abscheider (4) erhält und wobei der weitere Tank (6) mit einem Entkörnungskreislauf umfassend eine Sandentfernungseinheit (15) verbunden ist, die Sandentfernungseinheit (15) umfassend ein Ventil für Ascheauslass, sodass eine von Asche befreite Flüssigkeit periodisch aus dem weiteren Tank (6) in den ersten Tank (7) gegossen wird.

12. Anlage nach Claim 11, ferner umfassend:
f) einen Zerkleinerer (3), der zwischen dem Fermenter (1) und dem Abscheider (4) angeordnet ist.

13. Anlage nach Anspruch 11 oder 12, ferner umfassend mindestens eines von Folgenden:
g) einen ersten Wäscher (18) zur Biogaswäsche, worin mindestens ein Teil des in dem Fermenter (1) erzeugten Biogases (46) in Gegenstrom mit einem Gärreststrom (40, 62) gewaschen wird;
h) einen Abscheiderturm (23) zur Entfernung von organischen Stickstoffverbindungen aus einem Strom von gefiltertem Gärrest (40, 41), der in der Lage ist, einen abschließenden Strom von gefiltertem Gärrest (42) mit einem reduzierten Gehalt an Stickstoffverbindungen bereitzustellen; wobei der Abscheiderturm vorzugsweise mit einem zweiten Wäscher (25) zur Mineralisierung von organischen Stickstoffverbindungen verbunden ist, die in dem gasförmigen Strom umfassend Ammoniak (43) vorhanden sind, der aus dem Abscheiderturm (23) austritt, und wobei der gasförmige Strom (43) in Gegenstrom mit einer sauren Lösung (49) gebracht wird.

14. Anlage von Anspruch 13, umfassend
h) den Abscheiderturm (23) zur Entfernung von organischen Stickstoffverbindungen aus einem Strom von gefiltertem Gärrest (40, 41), der in der Lage ist, einen abschließenden Strom von gefiltertem Gärrest (42) mit einem reduzierten Gehalt an Stickstoffverbindungen bereitzustellen; und
i) einen Abschnitt für den Wärmeaustausch mittels eines Rohrbündelwärmetauschers, wobei der Zuführstrom (30) umfassend organisches Substrat und bakterielle Biomasse mit einem Überlaufstrom (32), der aus dem Fermenter (1) austritt, und mit dem abschließenden Strom aus filtriertem Gärrest (42) mit einem reduzierten Gehalt an Stickstoffverbindungen, der aus dem Abscheiderturm (23) austritt, in Kontakt gebracht wird.

## Revendications

1. Processus de production de biogaz, comprenant :
i) une phase de digestion anaérobie d'un substrat organique (30) dans un fermenteur (1), au moyen d'une biomasse bactérienne, formant une masse digérante dans ledit fermenteur (1), dans laquelle s'écoulent hors dudit fermenteur (1) : un flux de biogaz (46) se composant de biogaz et au moins un flux digérant (33) comprenant au moins une fraction de la masse digérante ;
ii) une phase de séparation du flux digérant (33), dans laquelle ledit flux digérant (33) est acheminé vers un séparateur (4), qui sépare un flux fibreux (35), comprenant la fraction fibreuse du flux digérant, et un flux liquide de digestat liquide (34) comprenant la fraction liquide du flux digérant et la biomasse bactérienne, et dans laquelle ledit séparateur est scellé de façon à réduire tout contact avec l'air ;
iii) une phase de recirculation du flux fibreux (35) vers le fermenteur (1), dans laquelle ledit flux fibreux (35) est sensiblement exempt d'oxygène ;
iv) une phase de concentration de la biomasse bactérienne contenue dans le flux liquide (34) par microfiltration, dans laquelle : ledit flux liquide (34) est acheminé périodiquement vers au moins un premier réservoir (7) maintenu dans les mêmes conditions d'anaérobiose à la même température que le fermenteur (1) depuis un réservoir supplémentaire (6), qui est placé entre le séparateur (4) et le premier réservoir (7) et est connecté à un circuit de dessablage comprenant une unité de dessablage (15), dans laquelle un flux de dessablage (37) est continuellement extrait du réservoir supplémentaire (6), acheminé vers le circuit de dessablage pour éliminer les cendres, puis renvoyé vers le réservoir supplémentaire (6), sans cendres ; ledit premier réservoir (7) étant connecté à un système de microfiltration (9) comprenant une membrane de microfiltration, via laquelle une partie du flux liquide (34) est micro-filtrée permettant d'obtenir un premier flux de digestat filtré (40) sensiblement exempt de biomasse bactérienne, et une partie du flux liquide (34) est retenue, permettant d'obtenir un flux retenu (39) comprenant la biomasse bactérienne concentrée qui rentre à nouveau dans le premier réservoir (7) ;
v) une phase de recirculation de la biomasse bactérienne vers le fermenteur (1), dans laquelle un flux de recirculation (31) comprenant la biomasse bactérienne s'écoule hors du premier réservoir (7) et est recirculé vers le fermenteur (1).

2. Processus selon la revendication 1, comprenant également une phase vi) de purification de biogaz, dans laquelle le premier flux de digestat filtré (40) sortant du système de microfiltration (9) est acheminé vers un premier épurateur (18) pour le nettoyage de biogaz , dans lequel il est mis à contre-courant avec au moins une partie du flux de biogaz (46) qui est produit par le fermenteur (1) ; dans laquelle sort dudit premier épurateur (18) un flux gazeux de biogaz purifié (47) et un second flux de digestat filtré (41) extérieur dont le pH est inférieur à celui du premier flux de digestat filtré (40) intérieur.

3. Processus selon la revendication 2, comprenant également une phase vii) d'élimination de composés nitrés du digestat, dans laquelle le second flux de digestat filtré (41) sortant du premier épurateur pour le nettoyage de biogaz (18) est acheminé vers une tour d'épuration (23) pour l'élimination de composés nitrés inorganiques, permettant d'obtenir un flux final de digestat filtré (42) qui sort de la tour d'épuration (23) avec une teneur réduite en azote, par rapport au second flux de digestat filtré (41) entrant, et un flux gazeux comprenant de l'ammoniac (43), qui s'écoule hors des ouvertures de la tour d'épuration (23).

4. Processus selon la revendication 3, dans lequel le flux gazeux comprenant de l'ammoniac (43), s'écoulant hors des orifices de la tour d'épuration (23), est acheminé vers un second épurateur (25) et est mis en contact à contre-courant avec une solution acide (49), permettant d'obtenir un flux azoté (44) comprenant des sels d'azote, sortant du second épurateur (25).

5. Processus selon la revendication 1, comprenant également une phase vi-b) d'élimination de composés nitrés du digestat, dans laquelle le premier flux de digestat filtré (40) sortant du système de microfiltration (9) est acheminé vers une tour d'épuration (23) pour l'élimination de composés nitrés inorganiques, permettant d'obtenir un flux de digestat d'effluent (62), qui sort de la tour d'épuration (23) avec une teneur réduite en azote par rapport au premier flux de digestat filtré (40) entrant et un flux gazeux comprenant de l'ammoniac (43).

6. Processus selon la revendication 5, comprenant également une phase vii-b) de purification de biogaz, dans laquelle le flux de digestat d'effluent (62) qui sort de la tour d'épuration (23) est acheminé vers un premier épurateur pour le nettoyage de biogaz (18), dans laquelle il est mis à contre-courant avec au moins une partie du flux de biogaz (46) qui est produit par le fermenteur (1), dans lequel ledit premier épurateur (18) évacue un flux gazeux de biogaz purifié (67) et un troisième flux de digestat (61) extérieur dont le pH est inférieur à celui du flux de digestat d'effluent (62).

7. Processus selon l'une quelconque des revendications 5 ou 6, dans lequel le flux gazeux comprenant de l'ammoniac (43), s'écoulant hors des ouvertures de la tour d'épuration (23), est acheminé vers un second épurateur (25) et est mis en contact à contre-courant avec une solution acide (49), permettant d'obtenir un flux azoté (44) comprenant des sels d'azote, sortant du second épurateur (25).

8. Processus selon l'une quelconque des revendications précédentes, comprenant une phase viii) dans laquelle un flux de fond (36) sort du fond du fermenteur (1) et est acheminé vers un épaississeur de sable (13), dans lequel une première portion du flux de fond (36) comprenant des cendres riches en phosphore et une seconde portion liquide, contenant la biomasse bactérienne purifiée des cendres, sont séparées, et dans lequel ladite seconde portion liquide est réintroduite dans le fermenteur (1).

9. Processus selon l'une quelconque des revendications précédentes, dans lequel en phase iii) ledit flux fibreux (35) comprenant la fraction fibreuse de la masse digérante est recirculé vers le fermenteur (1) au moyen d'une pompe de recirculation (5), dans lequel l'intérieur de ladite pompe de recirculation (5) est dans des conditions d'anaérobiose substantielle, dans lequel ladite pompe de recirculation (5) comprend une trémie de chargement qui reçoit le flux fibreux (35) provenant du séparateur et une chambre de dilution comprenant un liquide de dilution (48), et dans lequel le flux fibreux (35) est acheminé de la trémie de chargement vers la chambre de dilution, puis de cette dernière vers le fermenteur (1).

10. Processus selon l'une quelconque des revendications précédentes, comprenant une phase ix) dans laquelle un flux supérieur (32) comprenant une partie de la masse digérante est extrait du fermenteur (1) et dans laquelle la quantité de matière organique dans le fermenteur (1) est maintenue constante dans le temps en ajustant la proportion du flux supérieur (32) sur le flux digérant (33) sortant du fermenteur (1).

11. Usine de production de biogaz, comprenant :
a) au moins un fermenteur anaérobie (1) pour la production de biogaz, qui est alimenté par un flux d'alimentation (30) comprenant un substrat organique et une biomasse bactérienne, et d'où sort au moins un flux digérant (33), comprenant au moins une fraction d'une masse digérante ;
b) un séparateur (4) connecté audit fermenteur (1) et recevant le flux digérant (33) qui sort du fermenteur (1), ledit séparateur (4) étant apte à séparer au moins un flux liquide (34) comprenant une fraction liquide de digestat et au moins une partie de la biomasse organique, et un flux fibreux (35), comprenant une fraction fibreuse du produit digérant ;
c) un système de microfiltration (9) pour concentrer la biomasse bactérienne contenue dans le flux liquide (34), comprenant une membrane de micro-filtre via laquelle le flux liquide (34) est filtré, permettant ainsi d'obtenir un premier flux de digestat filtré (40), sensiblement exempt de biomasse bactérienne, et un flux retenu (39) comprenant une biomasse bactérienne concentrée ;
d) un premier réservoir (7) placé entre le séparateur (4) et le système de microfiltration (9), dans lequel le flux retenu (39) comprenant une biomasse bactérienne concentrée est envoyé et à partir duquel la biomasse bactérienne est périodiquement recirculée vers le fermenteur anaérobie (1) via un flux recirculant (31) ; dans laquelle ledit premier réservoir (7) est conservé à la même température que le fermenteur (1) dans des conditions d'anaérobiose et est connecté à une conduite de biogaz (46) provenant du fermenteur (1) ;
e) un réservoir supplémentaire (6) placé en aval du séparateur (4) et en amont du premier réservoir (7), ledit réservoir supplémentaire (6) étant maintenu à la même température que le fermenteur (1) et dans des conditions d'anaérobiose, dans laquelle ledit réservoir supplémentaire (6) reçoit le flux liquide (34) provenant du séparateur (4) et dans laquelle ledit réservoir supplémentaire (6) est connecté à un circuit de dessablage comprenant une unité de dessablage (15), ladite unité de dessablage (15) comprenant une vanne pour la décharge de cendres, de sorte qu'un liquide débarrassé de cendres est périodiquement versé dans le premier réservoir (7) depuis le réservoir supplémentaire (6).

12. Usine selon la revendication 11, comprenant également :
f) un déchiqueteur (3) placé entre ledit fermenteur (1) et ledit séparateur (4).

13. Usine selon la revendication 11 ou 12, comprenant également au moins l'un des éléments suivants :
g) un premier épurateur (18) pour le nettoyage de biogaz, dans lequel au moins une portion du biogaz (46) produit dans le fermenteur (1) est nettoyée à contre-courant avec un flux de digestat (40, 62) ;
h) une tour d'épuration (23) pour l'élimination de composés nitrés organiques d'un flux de digestat filtré (40, 41), apte à fournir un flux final de digestat filtré (42) **caractérisé par** une teneur réduite en composés azotés ; ladite tour d'épuration étant de préférence connecté à un second épurateur (25) pour la minéralisation de composés azotés organiques qui sont présents dans le flux gazeux comprenant de l'ammoniac (43) qui sort de la tour d'épuration (23), et dans lequel ledit flux gazeux (43) est mis à contre-courant avec une solution acide (49).

14. Usine selon la revendication 13, comprenant :
h) la tour d'épuration (23) pour l'élimination de composés nitrés organiques d'un flux de digestat filtré (40, 41), apte à fournir un flux final de digestat filtré (42) **caractérisé par** une teneur réduite en composés azotés ; et
i) une section pour un échange thermique au moyen d'un échangeur thermique à faisceau tubulaire, dans laquelle le flux d'alimentation (30) comprenant un substrat organique et une biomasse bactérienne est mis en contact avec un flux supérieur (32) sortant du fermenteur (1) et avec le flux final de digestat filtré (42) **caractérisé par** une teneur réduite en composés azotés qui sort de la tour d'épuration (23).
